# FASCICULE DE BREVET EUROPEEN

(11) **EP 0 672 425 B1**
(45) Date de publication et mention de la délivrance du brevet: **16.06.1999**
(21) Numéro de dépôt: 95490010.6
(22) Date de dépôt: 14.03.1995
(51) Int. Cl.: A61L 9/12

(54) **Diffuseur dynamique d'une substance telle qu'un parfum**
Dynamische Verdunstungsvorrichtung für Wirkstoffe wie Duftstoffe
Dynamic dispenser for a substance such as a perfume

(30) Priorité: 16.03.1994 FR 9403304
(43) Date de publication de la demande: 20.09.1995
(73) Titulaire: PRODIFA (S.A.R.L.), 59300 Valenciennes (FR)
(72) Inventeur: Lagneaux, Patrick, F-59264 Onnaing (FR); Peretti, Christian, F-59300 Valenciennes (FR)
(74) Mandataire: Hénnion, Jean-Claude

(56) Documents cités:
- WO-A-81/00051
- WO-A-94/06480
- CA-A- 1 174 101
- FR-A- 2 358 897
- GB-A- 1 454 040
- US-A- 4 944 898

## Description

La présente invention concerne un diffuseur de parfum ou plus généralement d'une substance qui diffuse naturellement dans l'atmosphère, par exemple désodorisant, insecticide, germicide, ... Plus particulièrement l'invention concerne un diffuseur dynamique de telles substances, c'est-à-dire un diffuseur qui est muni de moyens de brassage de l'air favorisant la diffusion naturelle de la substance et son évacuation dans l'atmosphère environnante.

On connaît par le document FR 2 358 897 un diffuseur dynamique qui comprend, dans un boîtier muni d'orifices d'évacuation de l'air, un ventilateur permettant de faire passer un courant d'air sur le produit supportant la substance à vaporiser.

Selon un mode préféré de réalisation décrit dans ce document antérieur, le diffuseur dynamique comporte une cartouche amovible qui est composée d'une part d'une pile cylindrique d'alimentation électrique du moteur entraînant en rotation le ventilateur et d'autre part d'un récipient annulaire monté autour de ladite pile cylindrique et contenant le matériau support de la substance à vaporiser. Ledit récipient annulaire est percé sur ses deux faces planes d'orifices de passage de l'air. Cette cartouche cylindrique est disposée coaxialement par rapport à l'axe de rotation du ventilateur et face à celui-ci de telle sorte que l'air créé par la rotation du ventilateur pénètre à l'intérieur du récipient, parcourt la surface du support de la substance à vaporiser et est évacué du boîtier. Le support de la substance à vaporiser est dans ce cas une bande poreuse imprégnée de ladite substance.

Dans le domaine des parfums, on connaît également des diffuseurs dynamiques de ce type dans lesquels l'élément parfumé est à l'état de gel ou de bloc solide. Le courant d'air provenant d'un ventilateur et parcourant la surface du gel ou du bloc accélère la diffusion naturelle du parfum.

Selon les constatations faites par le demandeur, ces diffuseurs dynamiques connus présentent un inconvénient qui consiste dans la diminution progressive de la concentration de l'air en substance parfumée au cours du temps. Ainsi dans la pièce où se trouve placé le diffuseur en question, l'utilisateur ne ressent pas la même sensation de parfum tout au long de l'utilisation dudit diffuseur.

S'agissant d'une substance à vaporiser telle qu'un insecticide, le diffuseur a une efficacité qui diminue dans le temps.

On a déjà proposé dans le document GB.1.454.O4O un diffuseur dans lequel la substance à diffusion naturelle est à l'état liquide. Le diffuseur en question comporte un récipient dont le fond est percé par un orifice qui donne dans une chambre contenant un matériau absorbant. Le liquide passe progressivement dans le matériau absorbant par gravité et par capillarité. La chambre est elle-même pourvue d'ouvertures latérales et un ventilateur est disposé face auxdites ouvertures en sorte qu'un flux d'air traverse la chambre et le matelas absorbant et se charge de la substance à diffusion naturelle.

Selon le demandeur ce diffuseur présente des inconvénients . En cas de saturation du matériau absorbant, la substance liquide goutte hors de la chambre et est perdue. La traversée du matériau absorbant par le flux d'air provoque une perte de charge importante, ce qui conduit à augmenter, pour un flux d'air donné, la puissance du moteur ou ventilateur. La chambre contenant la matériau absorbant est encombrante.

Le but que s'est fixé le demandeur est de proposer un diffuseur dynamique perfectionné qui pallie les inconvénients précités.

Il s'agit d'un diffuseur pour une substance liquide, telle qu'un parfum, à diffusion naturelle qui comporte un récipient pour ladite substance liquide et des moyens de brassage de l'air situé dans la zone de diffusion. De manière caractéristique,le récipient est fermé par une membrane imperméable au liquide et perméable au gaz; au moins une partie de ladite membrane constituant en position d'utilisation du diffuseur un flanc vertical du récipient; de plus il comporte des moyens de déplacement du récipient aptes à réaliser la rotation du récipient sur lui-même, selon un axe horizontal, provoquant la mise en contact au moins intermittente de la substance liquide avec la membrane constituant ledit flanc vertical.

Lors de la rotation sur lui-même du récipient le liquide qu'il contient se trouve toujours en contact avec au moins une portion du flanc vertical constitué de la membrane , quelle que soit la quantité de substance liquide se trouvant encore dans le récipient. Ainsi le déplacement du récipient permet d'humidifier constamment la membrane , même après une utilisation prolongée diminuant la quantité de substance liquide dans le récipient.

La membrane imperméable au liquide et perméable au gaz est une membrane poreuse dont les pores ont une taille appropriée pour permettre le passage d'un flux gazeux tout en empêchant le passage d'un liquide. Ce type de membrane, connu par ailleurs, est notamment en polypropylène. Avantageusement la face interne de ladite membrane est revêtue d'un matériau absorbant. Ceci a pour but d'obtenir une diffusion contrôlée en gardant constamment humide le matériau absorbant en contact avec la membrane.

De préférence, le récipient est de forme annulaire et les moyens de brassage sont placés au niveau de l'évidement central dudit récipient de manière à ce que la circulation d'air réalisée par lesdits moyens de brassage se fasse sur toute la surface du flanc vertical constitué par la membrane, depuis cet évidement central vers l'extérieur.

Avantageusement, selon cette même disposition, les moyens de brassage consistent en une hélice qui est montée sur une plaque circulaire tournante à la périphérie de laquelle est fixé le récipient annulaire, la membrane étant disposée radialement par rapport à la direction de refoulement de l'air provenant de l'hélice lors de la rotation de la plaque.

Ainsi, selon ce mode préféré de réalisation, la rotation du récipient et donc l'humidification continue de la membrane sont réalisées en même temps et à l'aide des mêmes moyens, que le brassage de l'air.

Avantageusement le diffuseur selon l'invention comporte un corps extérieur qui sert de logement à l'ensemble des éléments constituant ledit diffuseur à savoir le récipient annulaire , la plaque circulaire munie de l'hélice ainsi que les moyens d'entraînement en rotation de ladite plaque ; de plus ce corps extérieur est percé de deux jeux d'ouvertures, un premier jeu qui est dans une zone située à proximité de l'axe de rotation de la plaque, face à l'hélice, et un second jeu qui est dans une zone située radialement par rapport à ladite plaque. Ainsi on crée un flux d'air dont l'entrée dans le corps extérieur intervient par le premier jeu d'ouverture et dont la sortie intervient par le second jeu. Ce flux d'air passe obligatoirement le long de la surface de la membrane annulaire qui recouvre le récipient disposé en périphérie de la plaque circulaire.

De préférence dans ce cas le corps extérieur comporte un épaulement annulaire interne qui sépare les deux jeux d'ouverture et qui vient affleurer la surface supérieure de l'hélice lors de la rotation de la plaque circulaire sur elle-même. Cette disposition particulière apporte comme avantage de créer un passage obligé entre le premier et le second jeux d'ouvertures pour le flux d'air, passage qui est parcouru par les pales de l'hélice lors de la rotation de celle-ci. On a donc une efficacité beaucoup plus grande du brassage d'air par effet de turbine.

C'est un autre objet de l'invention que de proposer une cartouche autonome qui est destinée à être incorporée dans le diffuseur dynamique précité de substance, notamment de parfum. De manière caractéristique ladite cartouche est constituée :
a - d'une plaque circulaire comportant, selon son axe de rotation, des moyens de raccordement à l'arbre d'un moteur aptes à entraîner ladite plaque en rotation sur elle-même et sur une de ses faces des pales de brassage,
b - un récipient pour la substance à diffuser qui est constitué d'un corps creux annulaire, ayant une face ouverte, qui est fixé sur la périphérie de la plaque circulaire de telle sorte que la face ouverte soit dans le prolongement de ladite plaque,
c - une membrane imperméable au liquide et perméable au gaz qui est fixée sur la périphérie de la plaque par dessus la face ouverte du corps creux, après que la substance liquide à diffuser ait été placée à l'intérieur de celui-ci ,
d - un film obturateur, imperméable au liquide et au gaz, qui est fixé sur la périphérie de la plaque et qui recouvre ladite membrane.

Ainsi l'utilisateur qui dispose du diffuseur décrit précédemment n'a plus qu'à retirer le film obturateur qui a jusque là empêché toute diffusion de la substance, puis à placer la cartouche à l'intérieur du diffuseur de telle sorte que l'arbre du moteur d'entraînement contenu dans ledit diffuseur vienne se raccorder pour entraîner la plaque circulaire.

De préférence dans le cas d'une variante de réalisation d'un diffuseur avec un corps extérieur, ledit corps est constitué d'un boîtier auquel sont fixés les moyens d'entraînement en rotation de la cartouche et d'un couvercle pourvu des deux jeux d'ouvertures et de l'épaulement annulaire intérieur. Une fois que la cartouche a été placée dans le boîtier, il suffit à l'utilisateur de refermer le couvercle pour créer l'effet de turbine dont il a été question ci-dessus.

La présente invention sera mieux comprise à la lecture de la description qui va être faite du mode préféré de réalisation du diffuseur dynamique de parfum liquide, illustré par le dessin annexé dans lequel :
La figure 1 est une vue en coupe schématique d'une première variante d'un diffuseur dynamique de parfum liquide,
La figure 2 est une vue en coupe schématique d'une seconde variante d'un diffuseur dynamique, et
La figure 3 est une vue en plan de dessus du diffuseur de la seconde variante.

Le dispositif de l'invention a pour but de réaliser la diffusion d'une substance telle qu'un parfum, un désodorisant, un insecticide ou autre substance que l'on souhaite répandre dans l'atmosphère d'une pièce. Plus précisément ce dispositif est à diffusion dynamique en ce sens que la diffusion naturelle est combinée à un brassage de l'air.

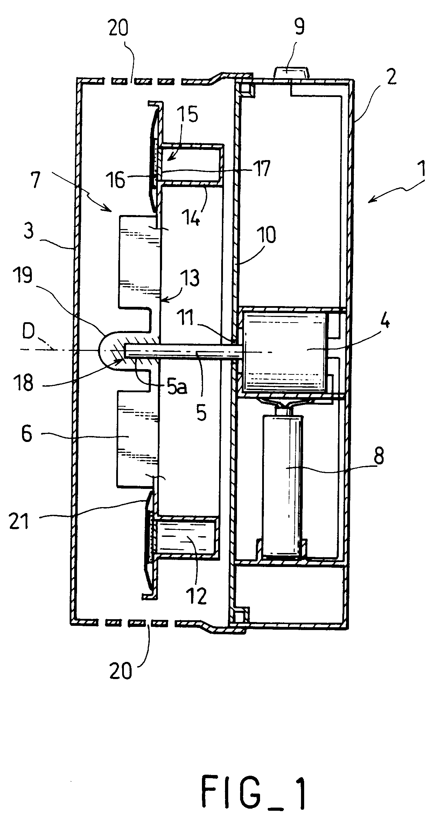

Dans l'exemple illustré à la figure 1, le dispositif est un diffuseur dynamique 1 de parfum qui se trouve à l'état liquide.

Le diffuseur 1 est composé d'un boîtier 2 fermé par un couvercle 3 dans lequel sont logés d'une part les éléments réalisant le brassage de l'air et d'autre part les éléments permettant la diffusion du parfum liquide.

Les éléments de brassage de l'air sont constitués d'un moteur 4 dont le corps est fixé latéralement dans la partie médiane du boîtier 2 et dont l'arbre de rotation 5 est disposé axialement dans le couvercle 3, ainsi qu'une hélice 6 qui fait partie d'une cartouche 7 amovible qui sera décrite ci-après.

Le moteur 4 est alimenté par pile 8 logée dans le boîtier 2 et commandée par un interrupteur 9 placé sur l'extérieur du boîtier 2. Bien sûr , cet interrupteur pourrait être placé à l'intérieur du boîtier pour des raisons esthétiques ou pour éviter son utilisation intempestive par une personne non habilitée.

Le boîtier 2 est fermé par une plaque d'obturation 10, emboîtable sur le boîtier 2 et comportant un orifice central 11 de passage pour l'arbre de rotation 5.

La cartouche 7 qui est destinée à être entraînée en rotation par l'arbre 5 est une cartouche amovible, jetable après usage, c'est à dire une fois que tout le parfum à l'état liquide 12 a été diffusé.

Cette cartouche 7 comporte, montés sur une plaque circulaire 13, d'une part le récipient 14 contenant le parfum 12 à l'état liquide et d'autre part l'hélice 6.

L'hélice 6 est constituée de pales, incluses dans deux cercles concentriques, par rapport à l'axe D de rotation de la cartouche 7. Le récipient 14 est un corps creux de forme annulaire, et de section transversale sensiblement rectangulaire, qui est disposé sur l'autre face de la plaque 13 par rapport à l'hélice 6. Le récipient 14 n'est fermé que sur trois de ses faces, la face ouverte 15 étant recouverte d'une membrane 16 qui est à la fois imperméable au liquide et perméable au gaz. Il s'agit par exemple d'une feuille de polypropylène microporeuse. Cette membrane 16 est fixée sur la plaque circulaire 13, de part et d'autre du récipient 14 par thermoscellage, thermosoudage thermique ou par ultrasons. Le thermosoudage peut être réalisé à vide ; dans ce cas , le remplissage du récipient peut intervenir par injection après thermosoudage.

De préférence la membrane 16 est revêtue, sur sa face interne, tournée vers l'intérieur du récipient 14 d'un matériau absorbant 17.

Comme cela apparaît sur la figure, le récipient 14 est disposé concentriquement par rapport à l'axe de rotation D de la cartouche 7, au-delà de l'hélice 6.

L'adaptation de la cartouche 7 sur l'arbre de rotation 5 se fait par emmanchement de l'extrémité 5a dudit arbre dans un logement 18 pratiqué dans un renflement 19 axial de la plaque 13.

De préférence le boîtier 2 et son couvercle 3 sont de forme sensiblement cylindrique et le couvercle 3 possède des orifices 20 de passage de l'air sur toute sa périphérie annulaire.

Pendant son stockage la cartouche 7 comporte également un film d'obturation 21 qui recouvre hermétiquement la membrane 16, étant fixé sur la plaque circulaire 13 de part et d'autre de ladite membrane 16. Il s'agit par exemple d'une feuille composite d'aluminium et de polyéthylène. Le polyéthylène empêche toute fuite du parfum à l'état liquide qui se trouve dans le récipient 14, tandis que l'aluminium, imperméable au gaz, évite toute déperdition de parfum. La fixation de ce film 21 est obtenue par thermoscellage, thermosoudage ultra-sons ou thermique.

Le fonctionnement du diffuseur est le suivant. L'utilisateur commence par décapsuler la cartouche 7 en retirant le film obturateur 21 de dessus de la membrane 16. Ensuite, le boîtier 3 étant ouvert, il emmanche la cartouche 7 sur l'arbre de rotation 5, enclenchant l'extrémité libre 5a dans le logement 18, dont la forme est adaptée pour recevoir cette extrémité 5a de l'arbre 5.

La cartouche 7 est alors en position d'utilisation et l'utilisateur peut refermer le couvercle 3. Grâce à l'interrupteur 9, il commande la mise en marche du moteur 4 et l'entraînement en rotation de l'arbre 5. La cartouche 7 tourne alors sur elle-même, par rapport à l'axe D horizontal.

Au cours de cette rotation, le parfum 12 à l'état liquide qui se trouve dans le logement 14 vient constamment en contact avec la membrane 16 imperméable au liquide et perméable au gaz qui constitue l'un des flancs latéraux du logement 14. Grâce à ce contact permanent le parfum peut diffuser naturellement à travers la membrane 16.

De plus lors de la rotation de la cartouche 7, l'hélice 6 provoque un déplacement de l'air depuis l'axe D jusque la périphérie de la plaque 13. La disposition selon laquelle l'hélice 6 est constituée de pales, incluses dans deux cercles concentriques, permet de réaliser un mouvement homogène et continu de l'air sur toute la périphérie de l'hélice, créant un flux qui balaie la zone qui est située en regard de la surface extérieure de la membrane 16, c'est à dire la zone dans laquelle se diffuse naturellement le parfum provenant du récipient 14. Le flux d'air passant par cette zone est projeté à travers les orifices 20 situés en périphérie du couvercle 3 et parvient de manière dynamique dans le local à parfumer.

Lors de l'utilisation du diffuseur 1, on constate bien sûr une diminution progressive du parfum à l'état liquide 12 dans le récipient 14 provenant de l'évaporation dudit parfum. Au fur et à mesure de cette diminution, le niveau de liquide 12 dans le logement 14 diminue, de sorte que progressivement seule la partie de la membrane 16 située, instantanément, dans le bas de la cartouche 16, est en contact avec le parfum 12 à l'état liquide. Cependant à chaque rotation du logement 14, la membrane 16 passe dans le liquide restant et est toujours plus ou moins imprégnée du parfum à l'état liquide 12. Cette imprégnation est encore favorisée grâce au matériau absorbant 17 recouvrant la surface interne de la membrane 16.

Ainsi il est possible d'utiliser l'intégralité du parfum liquide 12 contenu dans le récipient 14 tout en assurant une diffusion homogène et continue du parfum dans l'atmosphère.

En fonction du volume du local à parfumer, il sera possible de proposer des diffuseurs adaptés, avec une vitesse de rotation de l'arbre 5, une hauteur et une dimension des pales 6 fonction du flux d'air à déplacer ou encore avec un pilotage de la rotation par module électronique selon des cycles appropriés.

Le diffuseur 1 qui vient d'être décrit est alimenté par une pile, par exemple de 1,5 volt ou 9 volt, mais il est possible de prévoir une alimentation par des plaques solaires, ce qui permet d'éliminer tous les inconvénients qu'occasionne la mise en oeuvre de piles dans les lieux publics. Dans le cas d'alimentation du moteur par des plaques solaires, une régulation continue de la rotation du moteur pourra intervenir en masquant plus ou moins lesdites plaques solaires.

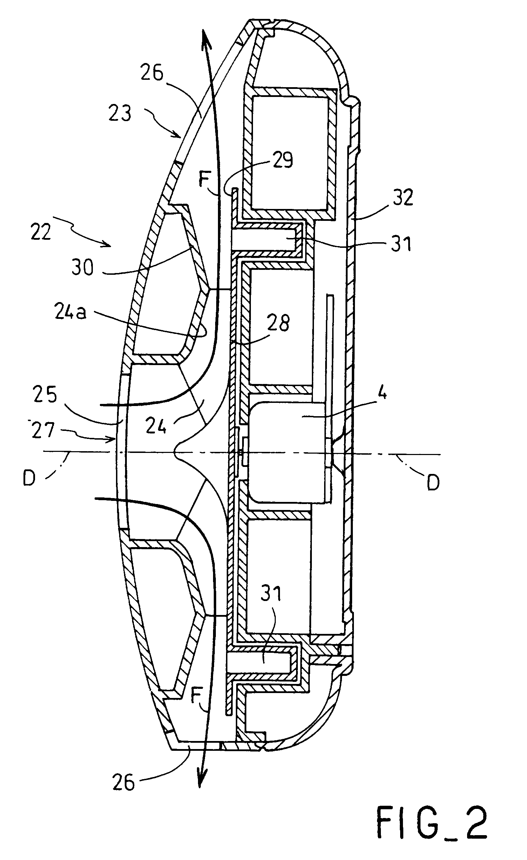

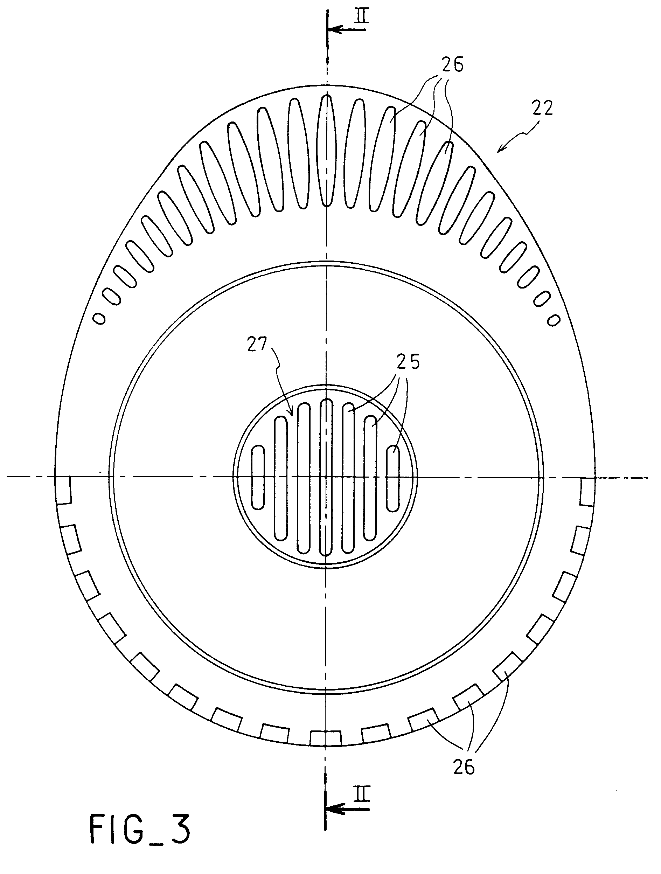

On a représenté aux figures 2 et 3 un second exemple de réalisation d'un diffuseur 22.

Ce diffuseur 22 se distingue principalement par rapport au premier exemple de réalisation, d'une part par la structure du couvercle 23 et d'autre part par la structure de l'hélice 24.

Comme cela apparaît clairement sur la figure 3, le couvercle 23 comporte deux jeux d'ouvertures 25,26. Le premier jeu d'ouvertures 25 est percé dans le couvercle 23 dans une zone 27 qui est située sensiblement en regard de l'axe D de rotation de la cartouche 28.

Le second jeu d'ouvertures 26 est situé dans une zone qui fait sensiblement le pourtour dudit couvercle 23 et qui est disposé radialement par rapport à la plaque circulaire 29 de la cartouche 28.

Ce même couvercle 23 comporte un épaulement interne 30 qui est de forme annulaire, symétrique par rapport à l'axe D, et qui vient affleurer sensiblement la face supérieure 24a de l'hélice 24, lors de la rotation de celle-ci autour de l'axe D.

Ainsi lors de la rotation sur elle-même de la cartouche 28 autour de l'axe D, il se crée un flux d'air à l'intérieur du couvercle 23, l'entrée d'air étant obtenue à travers le premier jeu d'ouvertures 25 et la sortie d'air étant obtenue par le second jeu d'ouvertures 26. Ce flux d'air, matérialisé par les flèches F sur la figure 2 , passe obligatoirement dans l'espace brassé par les pâles de l'hélice 24 et délimité entre la plaque circulaire 29 et l'épaulement annulaire 30. Ce flux d'air passe obligatoirement le long de la membrane, non représentée, recouvrant le récipient annulaire 31 contenant le parfum. Avec une hélice 24 munie de pales droites , on a obtenu d'excellents résultats sur le plan de l'efficacité aérodynamique.

Dans le second exemple de réalisation ,le diffuseur 22 n'a pas une forme circulaire mais une forme ovoïde, la pile d'alimentation du moteur étant logée dans la partie extrême supérieure du boîtier 32. Ceci permet d'avoir un diffuseur de très faible encombrement et présentant une forme extérieure tout-à-fait esthétique, comme cela apparaît à la figure 3. On comprend que pour avoir une répartition homogène du flux d'air, il importe que la surface des ouvertures 26 localisées dans la partie haute du boîtier soit égale à la surface des ouvertures 26 localisées dans la partie basse du même boîtier.

La présente invention n'est pas limitée aux modes de réalisation qui viennent d'être décrits à titre d'exemples non exhaustifs. On pourrait en particulier prévoir un système de bague scellé au moteur et trouvant son prolongement femelle dans la cartouche, ce qui permettrait d'éviter le retrait de ladite cartouche par tout utilisateur ne possédant pas le système adéquat. On pourrait prévoir également l'impossibilité d'adapter une autre cartouche.

## Revendications

1. Diffuseur dynamique pour une substance liquide, telle qu'un parfum, à diffusion naturelle, comportant un récipient pour la substance liquide et des moyens de brassage de l'air situés dans la zone de diffusion, caractérisé en ce que le récipient est fermé par une membrane imperméable au liquide et perméable au gaz, au moins une partie de ladite membrane constituant en position d'utilisation du diffuseur un flanc vertical du récipient, et en ce qu'il comporte des moyens de déplacement du récipient aptes à réaliser la rotation du récipient sur lui-même selon un axe horizontal, provoquant la mise en contact au moins intermittente de la substance liquide avec la membrane constituant ledit flanc vertical.

2. Diffuseur selon la revendication 1 caractérisé en ce que la face interne de la membrane , constituant le flanc vertical du récipient, est revêtue d'un matériau absorbant.

3. Diffuseur selon l'une des revendications 1 ou 2 caractérisé en ce que le récipient (14) est de forme annulaire et les moyens de brassage sont placés au niveau de l'évidement central dudit récipient de manière à ce que la circulation d'air réalisée par lesdits moyens de brassage se fasse sur toute la surface du flanc vertical constitué par la membrane, depuis cet évidement central vers l'extérieur.

4. Diffuseur selon la revendication 3 caractérisé en ce que les moyens de brassage consistent en une hélice (6) qui est montée sur une plaque circulaire (13) tournante, à la périphérie de laquelle est fixé le récipient annulaire (14), la membrane (16) étant disposée radialement par rapport à la direction de refoulement de l'air provenant de l'hélice (6) lors de la rotation de la plaque (13).

5. Diffuseur selon la revendication 4 caractérisé en ce qu'il comporte un corps extérieur contenant le récipient annulaire , la plaque circulaire munie de l'hélice et les moyens d'entraînement en rotation de ladite plaque et en ce que ledit corps extérieur est percé de deux jeux d'ouvertures 25, 26 , le premier jeu étant dans une zone située à proximité de l'axe de rotation D de la plaque circulaire 29, face à l'hélice 24 et le second jeu étant dans une zone située radialement par rapport à ladite plaque 29.

6. Diffuseur selon la revendication 5 caractérisé en ce que le corps extérieur comporte un épaulement annulaire interne 3O séparant les deux jeux d'ouvertures 25,26 et venant affleurer la surface supérieure 24a de l'hélice 24 lors de la rotation de la plaque 29.

7. Diffuseur selon la revendication 6 caractérisé en ce que le corps extérieur est composé d'un boîtier 32 et d'un couvercle 23, ledit couvercle 23 étant pourvue des deux jeux d'ouvertures 25,26 et de l'épaulement annulaire interne 3O.

8. Cartouche autonome qui est destinée à être incorporée dans le diffuseur dynamique de substance, notamment de parfum, selon l'une des revendications 4 ou 7 caractérisée en ce qu'elle est constituée :
a - d'une plaque circulaire (13) comportant, selon son axe de rotation (D), des moyens de raccordement (18) à l'arbre (5) d'un moteur (4) aptes à entraîner ladite plaque (13) en rotation sur elle-même et sur une de ses faces des pales (6) de brassage,
b - un récipient (14) pour la substance liquide (12) à diffuser qui est constitué d'un corps creux annulaire, ayant une face ouverte (15), qui est fixé sur la périphérie de la plaque circulaire (13) de telle sorte que la face ouverte (15) soit dans le prolongement de ladite plaque (13),
c - une membrane (16) imperméable au liquide et perméable au gaz qui est fixée sur la périphérie de la plaque (13) par-dessus la face ouverte (15) du corps creux, après que la substance liquide à diffuser ait été placée à l'intérieur de celui-ci ,
d - un film obturateur (21), imperméable au liquide et au gaz, qui est fixé sur la périphérie de la plaque (13) et qui recouvre ladite membrane (16).

## Claims

1. A dynamic diffuser for a naturally diffusing liquid substance such as a perfume, the diffuser comprising a receptacle for the liquid substance and air-flow generating means situated in the diffusion zone, the diffuser being characterised in that the receptacle is closed by a membrane that is impermeable to liquid and permeable to gas, at least a portion of said membrane constituting, in the position of use of the diffuser, a vertical flank of the receptacle, and in that it includes means for moving the receptacle so as to cause it to revolve about a horizontal axis, thereby causing the liquid substance to be put at least intermittently into contact with the membrane constituting said vertical flank.

2. A diffuser according to claim 1, characterised in that the inside face of the membrane constituting the vertical flank of the receptacle is covered in an absorbent material.

3. A diffuser according to one of claims 1 or 2, characterised in that the receptacle (14) is annular in shape, and the air-flow generating means are placed at the central opening of said receptacle in such a manner that the flow of air generated by said air-flow generating means takes place over the entire area of the vertical flank constituted by the membrane, outwardly from said central opening.

4. A diffuser according to claim 3, characterised in that the air-flow generating means comprise an impeller (6) mounted on a rotary circular plate (13), the annular receptacle (14) being fixed at the periphery thereof, the membrane (16) being disposed radially relative to the direction in which air is delivered by the impeller (6) while the plate (13) is rotating.

5. A diffuser according to claim 4, characterised in that it includes an outer body containing the annular receptacle, the circular plate carrying the impeller, and the means for rotating said plate, and in that said outer body is pierced by two sets of openings (25, 26), the first set being in a zone situated close to the axis of rotation (D) of the circular plate (29), facing the impeller (24), and the second set lying in a zone situated radially relative to said plate (29).

6. A diffuser according to claim 5, characterised in that the outer body includes an internal annular shoulder (30) separating the two sets of openings (25, 26) and flush with the top surface (24a) of the impeller (24) during rotation of the plate (29).

7. A diffuser according to claim 6, characterised in that the outer body is constituted by a housing (32) and by a cover (23), said cover (23) being provided with the two sets of openings (25, 26) and with the internal annular shoulder (30).

8. A self-contained cartridge for incorporating in the dynamic diffuser a substance, in particular a perfume, according to one of claims 4 or 7, and characterised in that it is constituted by :
a) a circular plate (13) including, on its axis of rotation (D), means (18) for coupling to the shaft (5) of a motor (4) suitable for causing said plate (13) to revolve, and also including, on one of its faces, blades (6) for generating a flow of air ;
b) a receptacle (14) for the liquid substance (12) to be diffused, which receptacle is constituted by an annular hollow body having an open face (15) and disposed at the periphery of the circular plate (13) in such a manner that the open face (15) is flush with said plate (13) ;
c) a membrane (16) that is impermeable to liquid and permeable to gas, which membrane is fixed at the periphery of the plate (13) over the open face (15) of the hollow body after the liquid substance to be diffused has been placed therein ; and
d) a closure film (21) that is impermeable to liquid and to gas, and that is fixed on the periphery of the plate (13) so as to cover said membrane (16).

## Patentansprüche

1. Dynamischer Diffusor für eine flüssige Substanz, wie etwa einen Duftstoff, mit natürlicher Diffusion, mit einem Behälter für die flüssige Substanz sowie mit Mitteln zum Umwälzen von Luft, die in der Diffusionszone gelegen sind, dadurch gekennzeichnet, daß der Behälter durch eine für Flüssigkeit undurchlässige und für Gas durchlässige Membran verschlossen ist, wobei mindestens ein Teil der genannten Membran in der Benutzungslage des Diffusors eine vertikale Flanke des Behälters bildet, und daß er Mittel zum Verlagern des Behälters aufweist, die dazu eingerichtet sind, eine Drehung des Behälters um sich selbst um eine horizontale Achse durchzuführen, die mindestens intermittierend die Berührung der flüssigen Substanz mit der Membran hervorruft, die die genannte vertikale Flanke bildet.

2. Diffusor nach Anspruch 1, dadurch gekennzeichnet, daß die innere Stirnfläche der Membran, die die vertikale Flanke des Behälters bildet, mit einem absorbierenden Material verkleidet ist.

3. Diffusor nach einem der Ansprüche 1 oder 2, dadurch gekennzeichnet, daß der Behälter (14) ringförmig ist und die Umwälzungsmittel auf Höhe der mittigen Aussparung des genannten Behälters derart angeordnet sind, daß der Luftumlauf, der durch die genannten Umwälzungsmittel hergestellt wird, auf der gesamten Oberfläche der vertikalen Flanke erfolgt, die von der Membran gebildet ist, und zwar von dieser mittigen Aussparung aus nach außen hin.

4. Diffusor nach Anspruch 3, dadurch gekennzeichnet, daß die Umwälzmittel eine Wendel bzw. einen Propeller (6) bilden, die bzw. der auf einer kreisförmigen, sich drehenden Platte (13) angebracht ist, an deren Umfang der ringförmige Behälter (14) befestigt ist, wobei die Membran (16) in Bezug auf die Förderrichtung der Luft, die von der Wendel (6) während der Drehung der Platte (13) herkommt, radial angeordnet ist.

5. Diffusor nach Anspruch 4, dadurch gekennzeichgnet, daß er einen äußeren Körper aufweist, der den ringförmigen Behälter, die kreisförmige Platte, die mit der Wendel versehen ist, und Drehantriebsmittel der genannten Platte aufnimmt, und daß der genannte äußere äußere Körper von zwei Gruppen von Öffnungen 25, 26 durchbrochen ist, wobei die erste Gruppe in einer Zone vorliegt, die in der Nähe der Drehachse D der kreisförmigen Platte 29 gelegen ist und der Wendel 24 gegenüberliegt, und die zweite Platte in einer Zone vorliegt, die in Bezug auf die genannte Platte 29 radial gelegen ist.

6. Diffusor nach Anspruch 5, dadurch gekennzeichnet, daß der äußere Körper eine ringförmige Innenschulter 30 aufweist, die die beiden Gruppen von Öffnungen 25, 26 trennt und mit der oberen Fläche 24a der Wendel 24 während der Drehung der Platte 29 fluchtet.

7. Diffusor nach Anspruch 6, dadurch gekennzeichnet, daß der äußere Körper aus einem Gehäuse 32 und einem Deckel 23 zusammengesetzt ist, und daß der genannte Deckel 23 mit den beiden Gruppen von Öffnungen 25, 26 und der inneren Ringschulter 30 versehen ist.

8. Selbständige Patrone, die dazu bestimmt ist, in den dynamischen Diffusor für Substanz, insbesondere für Duftstoff, nach einem der Ansprüche 4 oder 7, eingesetzt zu werden, dadurch gekennzeichnet, daß sie aus den folgenden Merkmalen gebildet ist:
a - aus einer kreisförmigen Platte (13), die längs ihrer Drehachse (D) Mittel zur Verbindung (18) mit der Welle (5) eines Motors (4) aufweist, die dazu eingerichtet sind, die genannten Platte (13) zur Drehung um sich selbst anzutreiben und auf einer ihrer Flächen Umwälz-Luftschaufeln (6) aufweist,
b - aus einem Behälter (14) für die flüssige Substanz (12), die zu diffundieren ist, der aus einem ringförmigen Hohlkörper mit einer offenen Stirnfläche (15) gebildet ist, der auf dem Umfang der kreisförmigen Platte (13) derart befestigt ist, daß die offene Fläche (15) in der Verlängerung der genannten Platte (13) liegt,
c - aus einer Membran (16), die für Flüssigkeit undurchlässig und für Gas durchlässig ist und die auf dem Umfang der Platte (13) über die offene Stirnfläche (15) des Hohlkörpers hinweggehend befestigt ist, nachdem die flüssige, zu diffundierende Substanz im Inneren dessen angeordnet wurde, und
d - aus einer Abdeckfolie (21), die für Flüssigkeit und Gas undurchlässig ist, auf dem Umfang der Platte (13) befestigt ist und die genannte Membran (16) abdeckt.
